# FASCICULE DE BREVET EUROPEEN

(11) **EP 0 707 477 B1**
(45) Date de publication et mention de la délivrance du brevet: **17.11.1999**
(21) Numéro de dépôt: 94921698.0
(22) Date de dépôt: 07.07.1994
(51) Int. Cl.: A61K 31/415

(54) **UTILISATION DE L'EFAROXAN ET DE SES DERIVES POUR LA FABRICATION DE MEDICAMENTS DESTINES AU TRAITEMENT DES MALADIES NEURODEGENERATIVES**
Verwendung von Efaroxan und dessen Derivaten zur Herstellung eines Arzneimittels zur Behandlung neurogenerativer Erkrankungen
USE OF EFAXORAN AND ITS DERIVATIVES FOR THE PRODUCTION OF DRUGS FOR TREATING NEURODEGENERATIVE DISEASES

(30) Priorité: 09.07.1993 FR 9308497
(43) Date de publication de la demande: 24.04.1996
(73) Titulaire: PIERRE FABRE MEDICAMENT, 92100 Boulogne (FR)
(72) Inventeur: COLPAERT, Francis, F-81100 Castres (FR); BRILEY, Michael, 81100 Castres (FR); IMBERT, Thierry, F-81290 Viviers-les-Montagnes (FR)
(74) Mandataire: Ahner, Francis
(86) Numéro de dépôt international: FR9400841
(87) Numéro de publication internationale: WO9501791

(56) Documents cités:
- EP-A- 0 071 368
- EP-A- 0 486 385
- US-A- 4 855 308
- J.MED.CHEM., vol.27, no.5, 1984 pages 570 - 576 C.B.CHAPLEO ET AL. 'Alpha-Adrenoreceptor Reagents. 2. Effects of Modification of the 1,4-Benzodioxan Ring System on alpha-Adrenoreceptor Activity'
- ARCH.INT.PHARMACODYN.THER., vol.277, no.2, 1985 pages 180 - 191 G.JOLY ET AL. 'Antagonistic Effects of S9871 or (imidazolinyl-2)-2-dihydro 2,3 benzofurane and its Stereoisomers on Some Central and Peripheral Actions of alpha2-Agonists'
- PSYCHOPHARMACOLOGY, vol.89, no.4, 1986 page S31 S.J.SARA ET AL. 'ENHANCEMENT OF COGNITIVE FUNCTION IN THE RAT BY ALPHA2 ANTAGONISTS OR ELECTRICAL STIMULATION OF THE LOCUS COERULEUS'
- BEHAV.NEURAL BIOL., vol.51, no.3, Mai 1989 pages 401 - 411 S.J.SARA ET AL. 'Idazoxan, an alpha2-Antagonist, Facilitates Memory Retrieval in the Rat'
- J.PSYCHOPHARMACOL., vol.4, no.2, 1990 pages 90 - 99 S.L.DICKINSON ET AL. 'Specific alpha2-adrenoceptor antagonists induce behavioural activation in the rat'
- NEUROCHEM.INT., vol.18, no.1, 1991 pages 137 - 140 H.DE VOS ET AL. 'EFAROXAN (RX 821037) IS A POTENT AND SELECTIVE alpha2-ADRENOCEPTOR ANTAGONIST IN HUMAN FRONTAL CORTEX MEMBRANES'

## Description

La présente invention a pour objet l'utilisation de l'Efaroxan et de ses dérivés pour obtenir un médicament neuroprotecteur, destiné au traitement de la démence sénile du type Alzheimer, du syndrome pré-Alzheimer, de la paralysie supra nucléaire progressive et d'autres maladies neurodégénératives.

La maladie d'Alzheimer est une maladie neurodégénérative progressive affectant particulièrement mais non exclusivement le système cholinergique central (Nucleus basalis de Meynert) se traduisant par une perte des facultés cognitives, une perte des capacités intellectuelles, des troubles du comportement et de la personnalité.

Il n'existe pas actuellement de traitement satisfaisant ni pour le traitement des symptômes, ni pour ralentir sa progression. Le diagnostic de la maladie est difficile, et l'on ne peut jamais avoir la certitude de diagnostiquer la maladie d'Alzheimer en elle-même. C'est en particulier l'analyse anatomo-histologique pratiquée post-mortem par, entre autres, la mise en évidence de plaques séniles extraneuronales et d'enchevêtrements neurofibrillaires intraneuronaux, qui peut permettre de diagnostiquer sans ambiguité la maladie d'Alzheimer. Lui sont associées également une perte de corps cellulaires et une déplétion de neurotransmetteurs, en particulier l'acétylcholine. En l'absence de ces preuves histologiques et biochimiques, les caractéristiques cliniques conduisent à diagnostiquer une maladie neurodégénérative, de type pré-Alzheimer.

La "Paralysie Supranucléaire Progressive" (P.S.P.) est une maladie neurodégénérative évolutive, non familiale, apparaissant tardivement et impliquant des perturbations de plusieurs neurotransmetteurs. La P.S.P. est caractérisée par une démence avec une instabilité posturale, une rigidité, une bradykinésie associées, accompagnée par une ophtalmoplégie supranucléaire. Cette maladie apparaît chez 4% environ des patients atteints de Parkinsonisme. Il n'existe actuellement aucun traitement de cette maladie. Les thérapeutiques palliatives ou symptomatologiques n'ont pas d'effet satisfaisant.

Il est connu que l'Efaroxan, 2-(2-éthyl-2,3 dihydrobenzo-furanyl)-2-imidazoline, possède des propriétés antagonistes sur les récepteurs α₂-adrénergiques. Ce composé est décrit dans la demande de brevet GB-2 102 422 ainsi que son application thérapeutique en tant que médicament antidépresseur et antimigraineux.

Ce composé et ses dérivés sont également décrits dans la demande de brevet WO 92/05171 où est mise en évidence l'action de l'énantiomère lévogyre pour traiter le diabète, comme agent bloqueur des canaux potassiques.

Par ailleurs, l'activité de l'Efaroxan, en tant qu'α₂-antagoniste, dans les membranes du cortex frontal humain a été décrite pax H. de Vos et al., NEUROCHEM.INT., vol. 18, no. 1, 1991, pages 137-140, et l'amelioration des tâches de rappel et d'attention chez lerat, par l'administration d'Idazoxan a été décrite par S.J. SARA et al., BEHAV. NEURAL. BIOL., vol. 51 no. 3, Mai 1989, pages 401-411. Cependant, aucun de ces documents ne concerne l'utilisation de l'Efaroxan conformément à la présente invention.

La présente invention concerne l'utilisation de l'Efaroxan et ses dérivés pour la préparation d'un médicament destiné au traitement de la démence sénile du type Alzheimer, du syndrome pré-Alzheimer, de la paralysie supra nucléaire progressive et d'autres maladies neurodégénératives. Par Efaroxan et ses dérivés, on entend le composé de formule I 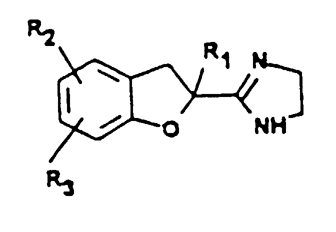 dans laquelle
R₁ représente un atome d'hydrogène, un radical alkyle linéaire ou ramifié en C₁-C₆,
R₂ représente un atome d'hydrogène, un groupe méthyle, chloro, bromo ou fluoro, et
R₃ représente un atome d'hydrogène, un groupe méthyle, hydroxy, methoxy, fluoro, chloro, ou bromo, et
   ses sels thérapeutiquement acceptables, son racémique ou ses isomères optiquement actifs.

D'une manière avantageuse, R₂ et R₃ représentent un atome d'hydrogène et R₁ représente un groupe éthyle, n-propyle ou i-propyle.

De préférence, le composé de formule générale I est choisi parmi les composés suivants :
2-(2-éthyl-2,3-dihydrobenzofuranyl)-2-imidazoline,
2-(2-n-propyle-2,3-dihydrobenzofuranyl)-2-imidazoline,
2-(2-i-propyle-2,3-dihydrobenzofuranyl)-2-imidazoline.

### ETUDE PHARMACOLOGIQUE

Une étude de l'activité de l'Efaroxan sur les fonctions mnésiques du rat a été réalisée selon les conditions décrites (P. CHOPIN et M. BRILEY, Psychopharmacology 106, 26 (1992)).

La scopolamine, un antagoniste central, administrée avant la première séance d'entraînement d'un comportement d'évitement passif, induit une amnésie dose-dépendante mesurable 48h plus tard lors d'une deuxième séance.

L'administration d'Efaroxan, 30 minutes avant la deuxième séance, diminue l'amnésie induite par la scopolamine, montrant ainsi une augmentation de l'activité mnésique par stimulation des fonctions de rappel.

Le tableau suivant montre l'effet de l'Efaroxan en présence de scopolamine, de façon dose dépendante, l'augmentation de temps que le rat met pour pénétrer d'un compartiment éclairé à un compartiment sombre où il reçoit une petite secousse électrique. Le pourcentage de l'effet est indiqué dans la 3e colonne.

| | **Dose mg/kg i.p.** | **latence d'entrée (sec)** | **% effet significatif** |
|---|---|---|---|
| Efaroxan | 0 | 28.6,±11.6 | |
| | 0.04 | 38.3±16.2 | +34 |
| | 0.16 | 56.0±15.3 | +96 |

Les résultats précédents montrent l'intérêt de l'Efaroxan à être utilisé en tant que médicament pour améliorer les fonctions de rappel et pour améliorer la symptomatologie de la maladie d'Alzheimer.

### ETUDE GALENIQUE

Les compositions pharmaceutiques sont administrées par voie orale sous forme de gélules ou de comprimés dosés de 1 à 100 mg de principe actif, plus particulièrement de 2,5 et 20 mg par gélule ou par voie intraveineuse sous forme de soluté injectable dosé de 0,1 à 10 mg d'Efaroxan.

### ETUDE CLINIQUE

L'Efaroxan a été administré à la dose de 2 à 20 mg par prise par jour pendant 6 mois, à des patients ayant manifesté des troubles de la mémoire, les symptômes de déficits cognitifs et de troubles comportementaux faisant suggérer un syndrome pré-Alzheimer. Les résultats sur la symptomatologie globale ont montré un bénéfice dans 30% des cas.

### ETUDE CLINIQUE P.S.P.

12 patients atteints de P.S.P. ont reçu de l'Efaroxan, à la dose de 2 mg 3 ou 4 fois par jour pendant 4 semaines en comparaison avec une période identique pendant laquelle un placebo était administré.

Après étude, la comparaison est en faveur de la période traitée par Efaroxan, pendant laquelle la symptomatologie observée est améliorée, de même que les scores des échelles usuelles ainsi que les tests cognitifs et d'humeur pour plus de 30% des cas.

## Revendications

1. Utilisation d'un composé de formule générale I 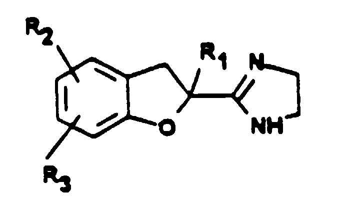 dans laquelle
R₁ représente un atome d'hydrogène, un radical alkyle linéaire ou ramifié en C₁-C₆,
R₂ représente un atome d'hydrogène, un groupe méthyle, chloro, bromo ou fluoro, et
R₃ représente un atome d'hydrogène, un groupe méthyle, hydroxy, methoxy, fluoro, chloro ou bromo, et
ses sels thérapeutiquement acceptables, son racémique ou ses isomères optiquement actifs,
pour la préparation d'un médicament destiné au traitement de la démence sénile du type Alzheimer, du syndrome pré-Alzheimer ou de la paralysie supra nucléaire progressive et d'autres maladies neurodégénératives.

2. Utilisation selon la revendication 1, caractérisée en ce que R₂ et R₃ représentent un atome d'hydrogène.

3. Utilisation selon l'une des revendications 1 ou 2, caractérisée en ce que R₁ représente un groupe éthyle, n-propyle ou i-propyle.

4. Utilisation selon la revendication 1, caractérisée en ce que le composé de formule générale I est choisi parmi les composés suivants :
2-(2-éthyl-2,3-dihydrobenzofuranyl)-2-imidazoline,
2-(2-n-propyle-2,3-dihydrobenzofuranyl)-2-imidazoline,
2-(2-i-propyle-2,3-dihydrobenzofuranyl)-2-imidazoline.

## Claims

1. Use of a compound of general formula I 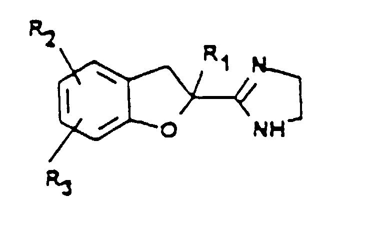 in which
R₁ represents a hydrogen atom or a linear or branched C₁-C₆ alkyl radical,
R₂ represents a hydrogen atom or a methyl, chloro, bromo or fluoro group, and
R₃ represents a hydrogen atom or a methyl, hydroxyl, methoxy, fluoro, chloro or bromo group, and
the therapeutically acceptable salts thereof, the racemic mixture thereof or the optically active isomers thereof,
for the preparation of a medicinal product intended for the treatment of Alzheimer-like senile dementia, pre-Alzheimer's syndrome or progressive supranuclear palsy and other neurodegenerative diseases.

2. Use according to Claim 1, characterized in that R₂ and R₃ represent a hydrogen atom.

3. Use according to either of Claims 1 or 2, characterized in that R₁ represents an ethyl, n-propyl or i-propyl group.

4. Use according to Claim 1, characterized in that the compound of general formula I is chosen from the following compounds:
2-(2-ethyl-2,3-dihydrobenzofuranyl)-2-imidazoline,
2-(2-n-propyl-2,3-dihydrobenzofuranyl)-2-imidazoline,
2-(2-i-propyl-2,3-dihydrobenzofuranyl)-2-imidazoline.

## Patentansprüche

1. Verwendung einer Verbindung der allgemeinen Formel I 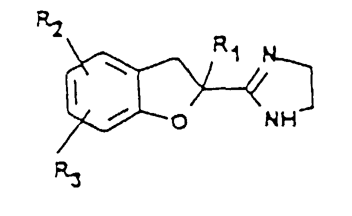 wobei
R₁ ein Wasserstoffatom, ein lineares oder verzweigtes C₁-C₆ Alkylradikal darstellt,
R₂ ein Wasserstoffatom, eine Methylgruppe, Chlor, Brom oder Fluor darstellt,
R₃ ein Wasserstoffatom, eine Methyl-, Hydroxy-, Methoxygruppe, Fluor, Chlor oder Brom darstellt und
ihrer therapeutisch verträglichen Salze, ihrer Racemate oder ihrer optisch aktiven Isomere
zur Herstellung eines Medikaments zur Behandlung der senilen Demenz vom Alzheimer-Typ, des Prä-Alzheimer-Syndroms oder der progressiven supranukleären Paralyse und weiterer neurodegenerativer Erkrankungen.

2. Verwendung nach Anspruch 1, dadurch charakterisiert, daß R₂ und R₃ ein Wasserstoffatom darstellen.

3. Verwendung nach einem der Ansprüche 1 oder 2, dadurch charakterisiert, daß R₁ eine Ethyl-, n-Propyl- oder i-Propylgruppe darstellt.

4. Verwendung nach Anspruch 1, dadurch charakterisiert, daß die Verbindung der allgemeinen Formel I aus den folgenden Verbindungen ausgewählt wird:
2-(2-Ethyl-2,3-dihydrobenzofuranyl)-2-imidazolin,
2-(2-n-Propyl -2,3-dihydrobenzofuranyl)-2-imidazolin
2-(2-i-Propyl-2,3-dihydrobenzofuranyl)-2-imidazolin.
